# EUROPEAN PATENT APPLICATION

(11) **EP 2 368 602 A1**
(43) Date of publication of application: **28.09.2011**
(21) Application number: 10177437.0
(22) Date of filing: 26.02.2004
(51) Int. Cl.: A61Q 15/00, A61K 8/92

(54) **Deodorant perfume composition comprising essential oils for masking an acid odour**

(30) Priority: 03.03.2003 JP 2003056017; 04.03.2003 JP 2003057462
(62) Divisional of application: 04714939.8
(71) Applicant: Takasago International Corporation, Tokyo-to 144-8721 (JP)
(72) Inventor: Sakurai, Kazutoshi, Tokyo Tokyo 144-8721 (JP); Sawano, Kiyohito, Kanagawa Kanagawa 254-0073 (JP); Yamazaki, Sadahiko, Kanagawa Kanagawa 254-0073 (JP); Hirano, Koji, Kanagawa Kanagawa 254-0073 (JP); Ogura, Miharu, Kanagawa Kanagawa 254-0073 (JP)
(74) Representative: Neidl-Stippler, Cornelia

(57) **Abstract**

The invention relates to a deodorant perfume composition enabled to mask an acid odor, said odor comprising one or more components selected from hydroxyalkynyl carboxylic acids having 5 to 8 carbon atoms and alkenyl carboxylic acids having 5 to 8 carbon atoms and one double bond comprising: (I) at least one kind or more selected from compounds or essential oils of which aroma is excellent in both of the masking and harmonizing effects to the acid odor; and at least one kind or more selected from compounds or essential oils of which aroma is excellent in the masking effect but neutral in the harmonizing effect to the acid odor. The application likewise refers to a deodorant composition and an external preparation composition, comprising the deodorant perfume composition and a method of inhibiting the acid body odor, by application of (A) at least one kind or more selected from compounds or essential oils that are excellent in both of masking and harmonizing effects; (B) at least one kind or more selected from compounds or essential oils that are excellent in the masking effect but neutral in the harmonizing effect (C) as needs arise, at least one kind or more selected from compounds or essential oils that exhibit neither the masking effect nor the harmonizing effect.

## Description

### Technical Field

The present invention relates to a perfume composition for inhibiting a body odor, in more detail a perfume composition for inhibiting a body odor that can efficiently mask and/or harmonize an acidic odor emitted from the body odor and the sweat odor, and a deodorant composition or external treatment composition including the same, and a novel body odor inhibiting method.

### Background Art

From a rise in the sense of cleanliness, care for, in particular, odors has become very important recently; among these, needs for eliminating odors emitted from human bodies, that is, body odors are particularly high. As the odors emitted from a human body, a scalp or head hair odor, a mouth odor, a sweat odor, an armpit odor, a foot odor and so on are known. On the other hand, there are also high demands for deodorizing a mouth odor and an excrement and urine odor due to sulfur compounds and so on, and a putrefaction odor due to particular components of decomposition products of organic compounds. Among these, the armpit odor is considered that the odor is generated by that secretions from sweat glands such as a sebaceous gland, an apocrine gland, an eccrine gland and so on are oxidized or subjected to an action of microbes. The odors such as the sweat odor, the foot odor and so on including the armpit odor generate unpleasant sensations in persons. Accordingly, there have so far been various devices to eliminate the odor. In view of these circumstances, products that intend to inhibit or reduce the body odors including the armpit odor and the sweat odor have been much provided to the market.

As methods of eliminating the odor, there have been known various methods such as a method of inhibiting the generation of the odor by inhibiting the generation of substances that generate odor materials, a method of inhibiting proliferation of microbes relevant to odor generation, and a method of masking an odor material with a particular perfume. In various kinds of commercially available products, at least any one of these methods for eliminating the odor is adopted. For illustration purpose, for instance, an antiperspirant that suppresses an amount of sweat that generates an odor material, a bacteriostat or microbicide that restrains proliferation of microbes, a masking agent due to a scent-emitting component and so on are compounded into commercial products in accordance with the object. In the antiperspirants, in almost all, an aluminum compound that is an astringent is used as a main component, usually aluminum chloride being used. Further, as the bacteriostat or microbicide, hexachlorophene and various kinds of quaternary ammonium chloride compounds are generally used. Furthermore, as the masking agent due to a scent-emitting component, there have been used various kinds of perfumes such as described in Japanese Patent Application Laid-open (JP-A) No. 6-179610, Japanese Patent Application National Publication (Laid-Open) No. 2002- 505264, and Japanese Patent Office: Published collection of well-known prior arts (perfume), part I, general of perfume (Jan. 29,1999) pp. 230 to 250.

When various kinds of products that have the masking or deodorizing effect to such odors are developed, it needs evaluating previously the masking effect and deodorizing effect to an odor of a substance or a composition to which application is intended and thereby carrying out screening. So far, in the case of the masking effect and the deodorizing effect to such odors being evaluated, it has been general to perform by using human bodies. However, in the case of the human body being used to evaluate, there are problems in that in addition to a large strain being forced on examinees, the examinees who take part in the evaluation can be secured with difficulty and furthermore from a human physiological viewpoint there is no warranty of the same phenomenon being always obtained. Furthermore, in the case of the evaluation being carried out with novel compounds and compositions, there is also a problem of safety. At this time, if a pseudo odor composition could be used in the evaluation, the novel compounds and compositions would be evaluated without paying attention to the problems as mentioned above.

From such viewpoints, pseudo human body odors for evaluating with simplicity the masking, harmonizing and deodorizing effect of the odor to particular odors such as the sweat odor, the armpit odor and so on have been looked for; hitherto, various human body odor components of such as a mouth odor, a scalp odor, an armpit odor, a foot odor, a sweat odor and so on have been studied. (see, for instance, Journal of Chemical Ecology, (USA) 1991,17 (7), pp. 1469 to 1492; Head Office of 46th TEAC Meeting (hosted by Chemical Society of Japan), 46th Discussion on perfume/Terpene and essential oil chemical, contents of lecture, 2002, pp. 124 to 126 ; and European Patent Application Laid-open No. 1,258, 531). When a pseudo body odor composition very close to an actual body odor can be obtained, it is obvious that while studying, as mentioned above, the safety of the novel substance or composition to human bodies on one hand, on the other hand, in parallel therewith, irrespective of the safety, the masking, harmonizing or deodorizing effect of the substance or composition to the armpit odor resultantly the sweat odor can be accurately, simply and efficiently evaluated, and thereby the substances and compositions that have the effect can be efficiently subjected to the screening.

On the basis of studies on these body odor components, studies for preparing model odors and proposals of model odors have been forwarded. Among the pseudo human body odors, as to the scalp odor, a model odor for evaluation comprising squalene, cholesterols and fatty acids as main components is proposed (JP-A Nos. 2001-220593 and 2001- 271088). On the other hand, as to the sweat odor, there have been mainly studied components that are generated when a sebaceous matter and higher fatty acids are oxidatively decomposed owing to indigenous bacteria in skin. However a pseudo odor more close to actual sweat odor, in particular, a peculiar odor secreted from the apocrine gland of such as armpits is disclosed in EP 1601338A1. The apocrine gland is present also in eyelids, external auditory meatus, the surroundings of anus, surroundings of nipple and so on other than the armpits.

On the other hand, in the deodorant products, various kinds of perfume compositions are blended ; however, it is a present state that as to perfume materials that can effectively mask the odor of armpits, the perfume materials having characteristics with sufficient satisfaction are not yet provided.

### Disclosure of Invention

In view of these circumstances, the present inventors analyzed components obtained by solvent extraction from a human armpit portion by use of gas chromatography, Olfactometry, mass spectrometry and so on and found this time a peak showing an odor characteristic to the armpit odor. Furthermore, by means of mass spectrometry analysis and chemical synthesis, the components were determined. So far, as the components of the sweat odor, various kinds of carboxylic acids including 3-methyl-2-hexenoic acid and acetic acid were reported. In addition, as a result of the research due to the present inventors, it was newly found that 3-hydroxy-3-methylhexanoic acid is contained in the components of the armpit odor and an enantiomer ratio of the hydroxycarboxylic acid, that is, a ratio of R-body to S-body of the enantiomers is 1: 3. The odor of the compound was an odor that suggests the armpit odor different from that of the so far known 3-methyl-2-hexenoic acid. As to the odor of the hydroxycarboxylic acid, a method of masking it with an odorous component has not yet been reported.

An object of the invention is to provide a perfume composition for inhibiting a body odor that can efficiently mask and/or harmonize a body odor and a sweat odor including an armpit odor that includes the odor components.

Furthermore, a still another object of the invention is to provide a perfume composition for inhibiting a body odor that can effectively mask and/or harmonize an acid odor emitted from the body odor and the sweat odor.

Still furthermore, a still another object of the invention is to provide a deodorant composition or an external treatment composition that includes a perfume composition for inhibiting a body odor that can effectively mask and/or harmonize an acid odor emitted from the body odor and the sweat odor.

Still furthermore, a still another object of the invention is to provide a deodorant composition or an external treatment composition that include a perfume composition for inhibiting a body odor that can efficiently mask and/or harmonize a sweat odor and an armpit odor.

Still furthermore, a still another object of the invention is to provide a method of inhibiting a body odor.

The present inventors carried out the screening to many single perfumes with the pseudo body odor compositions of EP 1601338 A1. As a result, it was found that some kinds of perfumes can efficiently mask and/or harmonize the acid odor such as the sweat odor and the armpit odor, that is, the acid odor selected from the aforementioned hydroxyalkynyl carboxylic acids having 5 to 8 carbon atoms and alkenyl carboxylic acids having 5 to 8 carbon atoms and one double bond, and thereby the present invention was achieved.

The present invention relates to deodorant perfume compositions for inhibiting a body odor according to the following items [1] to [4], an external preparation composition according to item [5] and methods of inhibiting a body odor according to items [6] and [7].
[1] A deodorant perfume composition enabled to mask an acid odor, comprising one or more components selected from hydroxyalkynyl carboxylic acids having 5 to 8 carbon atoms and alkenyl carboxylic acids having 5 to 8 carbon atoms and one double bond comprising:
   (I) at least one kind or more selected from compounds or essential oils of which aroma is excellent in both of the masking and harmonizing effects to the acid odor;
   (II) at least one kind or more selected from compounds or essential oils of which aroma is excellent in the masking effect but neutral in the harmonizing effect to the acid odor.
[2] The deodorant perfume composition as set forth in item [1], wherein the acid odor is 3-hydroxy-3- methylhexanoic acid and/or 3-methylhexenoic acid.
[3] The deodorant perfume composition as set forth in item [1], wherein the component (I) is contained by 40 % by weight or more, components (I) and (II) are contained by 60 % by weight or more in sum total; further components are contained by less than 40 % by weight.
[4] A deodorant composition, comprising the deodorant perfume composition as set forth in any of the items [1] to [3].
[5] An external preparation composition, comprising the deodorant perfume composition as set forth in any of the items [1] to [3].
[6] A method of inhibiting an acid body odor selected from hydroxyalkynyl carboxylic acids having 5 to 8 carbon atoms and alkenyl carboxylic acids having 5 to 8 carbon atoms and one double bond, by application of
   (A) at least one kind or more selected from compounds or essential oils that are excellent in both of masking and harmonizing effects;
   (B) at least one kind or more selected from compounds or essential oils that are excellent in the masking effect but neutral in the harmonizing effect.
   (C) as needs arise, at least one kind or more selected from compounds or essential oils that exhibit neither the masking effect nor the harmonizing effect.
[7] The method of inhibiting a body odor as set forth in item 6, wherein an acid odor is 3-hydroxy-3-methylhexanoic acid and/or 3-methylhexenoic acid.

In addition, the perfume compositions for inhibiting a body odor according to the present invention may be appropriately and arbitrarily mixed with other perfume compositions in accordance with the object. Accordingly, the perfume compositions for inhibiting a body odor of the present invention can be widely used in various kinds of blended perfumes.

### (1) Pseudo body odor composition

In the present invention, the pseudo body odor compositions of EP 1601338 A1 that are more close to an actual odor of armpits were used.

Carboxylic acids of component (A) in the pseudo body odor compositions of the invention are important in the reproduction of an "odor peculiar to the apocrine gland" that is a peculiar odor due to the apocrine gland excretions in the sweat odors. The "odor peculiar to apocrine gland" denotes one that is displayed with the apocrine gland excretions as a cause material and has the nature of strongly smelling as the body odor (odor of sweat, odor of armpits). The odor peculiar to the apocrine gland strongly and instantaneously smells particularly in crowded trains and at the time when a person moved from one room to another room and gives more unpleasant sensation to others. When the odor peculiar to the apocrine gland is not taken into consideration, the preparation of the odor close to the sweat odor and the armpit odor in particular cannot be achieved.

The component (A) of the pseudo body odor composition according to EP 1601338 A1 comprises at least one kind of hydroxyalkynyl carboxylic acids having 5 to 8 carbon atoms that is the component (A-1) and/or at least one kind of alkenyl carboxylic acids having 5 to 8 carbon atoms and one double bond that is the component (A-2).

The prepared pseudo body odor composition of EP 1601338 A1 is close to actual sweat odor, armpit odor and so on. Therefore, by use of the composition, accurate and efficient evaluations of the masking capability, the harmonizing effect or the deodorizing effect to the sweat odor, armpit odor and so on can be made. As a result, substances that have the masking capability, the harmonizing effect or the deodorizing effect to the odor of the sweat odor, armpit odor and so on can be efficiently screened.

When the pseudo body odor composition of EP 1601338 A1 for use in evaluation of the invention are used, the evaluation of the masking capability, the harmonizing effect or the deodorizing effect to the body odor, in particular, the sweat odor, the armpit odor and so on caused by human sweat can be easily performed by, for instance, adding a specimen to the pseudo body odor composition for use in evaluation of the invention or bringing a specimen into contact with the inventive composition, followed by smelling and comparing the odor with a odor of a body odor composition of the invention. As a specific method, a cup method may be used. In the evaluation of the odor, it is preferable to carry out by 1 to 20 expert panelists and general panelists. Further, the evaluation is preferably carried out by a 2 to 7 grades evaluation, and more preferably a 3 to 5 grades evaluation by expert panelists.

By use of the pseudo body odor composition for use in evaluation according to EP 1601338 A1, effects of masking, harmonizing or deodorizing the body odor, in particular, the sweat odor, the armpit odor and so on caused by human sweat can be easily evaluated, and substances having these effects can be accurately and efficiently screened. In addition, while sufficiently studying the safety on one hand, on the other hand, in parallel with the study of the safety, functional evaluation can be performed. Accordingly, efficient product development can be realized.

### (2) Deodorant perfume composition

A perfume composition for inhibiting a body odor of the invention that can effectively mask and/or harmonize an acid odor selected from hydroxyalkynyl carboxylic acids having 5 to 8 carbon atoms and alkenyl carboxylic acids that have 5 to 8 carbon atoms and one double bond that are emitted from body odor and the sweat odor (hereinafter, in some cases, referred to as"acid odor such as the sweat odor and armpit odor") typically includes, as components, (I) compounds or essential oils excellent in both of masking and harmonizing effects to the acid odor such as the sweat odor and the armpit odor (hereinafter, in some cases, referred to as "masking component"), (II) compounds or essential oils of which aroma is excellent in the masking effect but neutral in the harmonizing effect to the acid odor such as the sweat odor and the armpit odor (hereinafter, in some cases, referred to as "harmonizing neutral component"), and (III), as needs arise, compounds or essential oils of which aroma exhibits neither the masking nor harmonizing effects to the acid odor (hereinafter, in some cases, referred to as "balance component"). In the following, the compounds and essential oils contained in the respective components will be specifically explained. However, the respectively exemplified compounds and essential oils of components (I) to (III) described below are illustrated as only examples ; that is, the above components (I), (II) and (III) according to the invention are not restricted to the exemplified ones below.

### (I) Masking component

As the masking components that are components excellent in both of the masking effect and the harmonizing effect to the acid odor such as the sweat odor and the armpit odor, there are illustrated, for example, compounds and essential oils selected from the following respective groups of (I-1) to (I-7).

### (I-1) Alcohols

cis-3-Hexenol, phenylethyl alcohol, citronellol, 2- ethyl-4- (2, 2, 3-trimethyl-3-cyclopentene-1-yl)-2-butene-1-ol (Levosandol: manufactured by Takasago International Corp.), 2-methyl-4- (2, 2, 3-trimethyl-3-cyclopentene-1-yl)-2-butene-1-ol (Sandalmysore Core: manufactured by Kao Corp.), geraniol and borneol.

### (I-2) Esters and lactones

cis-3-Hexenyl acetate, styralyl acetate, allyl amyl glycolate, phenylethyl acetate, benzyl acetate, cis-3- hexenyl salicylate, 2-tert-butylcyclohexyl acetate (Verdox manufactured by Kao Corp. ), methyl dihydrojasmonate (Hedion: manufactured by Firminich, Inc. ), methyl benzoate, methyl phenyl acetate, ethyl acetoacetate, allyl caproate, ethyl-2-methyl butylate, ethyl-2-methyl valerate, allyl cyclohexyl oxyacetate (Cyclogalbanate: manufactured by Dragoco), methyl anthranilate, γ-undecalactone, γ-nonalactone, γ-decalactone, and coumarin.

### (I-3) Aldehydes and acetals

Dimethyl tetrahydrobenzaldehyde (Tripral: manufactured by IFF), ethyl vanillin, citronellal, hexanal, octanal, benzaldehyde, 2,6-dimethyl-5-heptenal (Melonal: manufactured by Givaudan), citral, trimethyl cyclohexene carbalehyde (Isocyclocitral : manufactured by IFF), 4-methoxybenzaldehyde, citral diethyl acetal (Citrathal: manufactured by Quest), acetaldehyde phenyl ethyl propylacetal and phenyl acetaldehyde dimethylacetal.

### (I-4) Ketones

Damascenone, methylionone soap, β-damascone, camphor, methylene tetramethyl heptanone (Koavone: manufactured by IFF), 1- (5, 5-dimethylcyclohexene-1-yl)-4-pentene-1-one (α-Dynascone : manufactured by Firminich), menthone and 1- carvone.

### (I-5) Ethers

Decahydro-3a, 6, 6, 9α-tetramethyldodecanhydronaphto [2, 1- b] furan (Ambroxan: manufactured by Henkel), 2-butyl-4, 6- dimethyl-dihydro-pyran (Vigorose: manufactured by Quest), p-cresyl methyl ether, phenyl acetaldehyde, rose oxide, and β-naphthol methyl ether.

### (I-6) Nitrogen-containing compounds

Isobutylquinoline, geranyl nitrile, citronellyl nitrile, and Hydoroxycitronellylidene methyl anthranilate (Aurantiol: manyfactured by Takasago International Corp.).

### (I-7) Essential oils

Lemon oil, lime oil, orange oil, rosemary oil, ylang-ylang oil, mandarin oil, eucalyptus oil, lavandin oil, geranium oil, chamomile oil, cardamom oil, citronella oil, elemi oil, cinnamon oil, petitgrain oil, taget oil, armoise oil, galbanum oil, peppermint oil, clary sage oil, clove bud oil, rose oil, basil oil, and spearmint oil.

Among these masking components, as components excellent in the masking effect to the sweat odor, in particular, to the armpit odor, there are illustrated;
(I-1) alcohols selected from cis-3-hexenol, phenylethyl alcohol, citronellol, 2-ethyl-4- (2, 2,3- trimethyl-3-cyclopentene-1-yl)-2-butene-1-ol (Levosandol : manufactured by Takasago International Corp. ), 2-methyl-4- (2,2, 3-trimethyl-3-cyclopentene-1-yl)-2-butene-1-ol (Sandalmysore Core: manufactured by Kao Corp. ), geraniol and borneol;
(I-2) esters and lactones selected from cis-3-hexenyl acetate, styralyl acetate, allyl amyl glycolate, phenylethyl acetate, benzyl acetate, cis-3-hexenyl salicylate, 2-tert-butylcyclohexyl acetate (Verdox : manufactured by Kao Corp. ), methyl dihydrojasmonate (Hedion: manufactured by Firminich, Inc. ), γ-undecalactone and coumarin;
(I-3) one kind or more aldehydes selected from dimethyl tetrahydrobenzaldehyde (Tripral: manufactured by IFF) and ethyl vanillin;
(I-4) ketones selected from damascenone, methylionone and β-damascone ;
(I-5) decahydro-3α, 6,6, 9α- tetramethyldodecanhydronaphto [2, 1-b] furan (Ambroxan: manufactured by Henkel) that is ethers ; and
(I-7) essential oils selected from lemon oil, lime oil, orange oil, rosemary oil, ylang-ylang oil, mandarin oil, eucalyptus oil, lavandin oil and geranium oil.

Each of these compounds or essential oils of the group (A) may be compounded singly or in combinations of two or more kinds as the component (I) (masking component) in the compositions for inhibiting a body odor of the invention. A compounding amount of each of these masking components in the perfume composition for inhibiting a body odor of the invention is appropriately selected in accordance with components being specifically selected, the masking effect to be invested for the sweat odor, the armpit odor and so on, and further other blending components; that is, it is not restricted to particular ones. In the present invention, by compounding the masking component, the excellent masking effect is exhibited for the sweat odor, in particular for the armpit odor. Thereby, the compositions for inhibiting a body odor of the invention that are useful for the deodorants, external preparations and so on including perfumeries can be provided.

### (II) Harmonizing neutral component

As the harmonizing neutral components that are compounds or essential oils of which fragrance is excellent in the masking effect but neutral in the harmonizing effect to the acid odor such as the sweat odor or the armpit odor, there are illustrated, for example, one or more kinds of compounds or essential oils selected from the following respective groups of (II-1) to (II-7).

### (II-1) Alcohols

Ethyl linalool, tetrahydromyrcenol, 4-methyl-3-decene- 5-ol (Undecavertol: manufactured by Givaudan), 9-decene-1-ol, p-tert-butylcyclohexanol and dimethyl octanol.

### (II-2) Esters

Allyl cyclohexyl propionate, terpinyl acetate, tricyclodecenyl acetate, citronellyl acetate, amyl salicylate, phenylethyl phenyl acetate, ethyl 3-methyl-3-phenyl glycidate, ethyl 2,2,6-trimethylcyclohexane carboxylate (Thesaron: manufactured by Takasago International Corp. ), trichloromethyl phenyl carbinyl acetate (rosephenone) and anisyl acetate.

### (II-3) Aldehydes

Heliotropine, p-tert-butyl-α-methyl hydrocinnamic aldehyde (Lilial: manufactured by Givaudan), 4(3)-(4-hydroxy-4-methylpentyl)-3-cyclohexene-1-carboxy aldehyde (Kovanol: manufactured by Takasago International Corp.), hydroxy citronellal, α-methyl-3, 4-methylene dioxyhydrocinnamic aldehyde (Helional: manufactured by IFF), cinnamic aldehyde, 2-methyl undecanal, 4-tricyclodecylene butanal, p-ethyl-α,α-dimethyl phenyl propyl aldehyde (Floralozone: manufactured by IFF) and 4-(4-methyl-3-pentenyl)-3-cyclohexene-1-carbaldehyde (Myrac aldehyde: manufactured by IFF).

### (II-4) Ketones or ketals

α-Damascone, δ-damascone, 6-acetyl-1,1,2,4,4,7- hexamethyl tetrahydro naphthalene (Tonalid: manufactured by PFW), allyl ionone, 4-tert-butyl-2,6-dimethylacetophenone, 5-cyclohexadecene-1-one (Ambretone: manufactured by Takasago International Corp.), and 3-methyl-2-pentyl-2-cyclopentene-1-one (dihydrojasmone), 2,4,6-trimethyl-4-phenyl-1,3-dioxane (Floropal: manufactured by Harman & Rimmer).

### (II-5) Ethers

Eugenol, diphenyl oxide and 4-methyl-2-(2- methylpropyl)tetrahydro-2H-4-pyranol (dihydro rose oxide).

### (II-6) Nitrogen-containing compounds

Tridecene-2-nitrile.

### (II-7) Essential oils

Cedar wood oil, juniperberry oil, clove leaf oil, thyme oil and nutmeg oil.

Among these harmonizing neutral components, as components excellent in the masking effect to the sweat odor, in particular, the armpit odor, there are illustrated,
(II-1) ethyl linalool in alcohols;
(II-2) esters selected from allyl cyclohexyl propionate, terpinyl acetate, tricyclodecenyl acetate, citronellyl acetate and amyl salicylate;
(II-3) aldehydes selected from heliotropine, p-tert-butyl-α-methyl hydrocinnamic aldehyde (Lilial : manufactured by Givaudan), 4(3)-(4-hydroxy-4-methylpentyl)-3-cyclohexene-1-carboxy aldehyde (Kovanol: manufactured by Takasago International Corp.), hydroxy citronellal and α-methyl-3,4-methylene dioxyhydro cinnamic aldehyde (Helional: manufactured by IFF);
(II-4) ketones and ketals selected from α-damascone, δ-damascone and 6-acetyl-1,1,2,4,4,7-hexamethyl tetrahydro naphthalene (Tonalid: manufactured by PFW);
(II-5) eugenol in ethers ; and
(II-7) cedar wood oil in essential oils.

The compounds or essential oils of the II groups each, may be compounded singly or in combinations of two or more kinds as the component (II) (harmonizing neutral component) into the compositions for inhibiting a body odor of the invention. A compounding amount of each of these masking components in the perfume compositions for inhibiting a body odor of the invention is appropriately selected in accordance with components being specifically selected, the masking effect to be invested for masking the sweat odor, the armpit odor and so on, and further other compounding components; that is, it is not restricted to particular ones. In the present invention, by compounding the harmonizing neutral component, the excellent masking effect is exhibited for the sweat odor, in particular, for the armpit odor. Thereby, the compositions for inhibiting a body odor of the invention that are useful for the deodorants, external preparations and so on including perfumeries can be provided.

### (III) Balance component

As the balance components that are made of compounds or essential oils that exhibit neither the masking nor harmonizing effects to the acid odor such as the sweat odor and the armpit odor, there are illustrated, for example, one or more kinds of compounds or essential oils selected from the following respective groups of (III-1) through (III-6). These balance components have an effect of harmonizing fragrance of blended perfume.

### (III-1) Alcohols and phenols

Dihydromyrcenol, isocamphyl cyclohexanol (Santalex: manufactured by Takasago International Corp.), dimethyl phenyl ethyl carbinol, 2-phenylpropanol, 2,2-dimethyl-3-(3-methylphenyl)propanol, 1-Menthol, cis-p-isopropylcyclohexyl methanol (Mayol: manufactured by Firmenich) α, α-dimethyl phenylethyl alcohol, 1-(2-tert-butylcyclohexyloxy)butane-2-ol (Ambercore: manufactured by Kao Corp. ), 1-(2, 2, 6- trimethylcyclohexyl)-3-hexanol (Ambestole: manufactured by Takasago International Corp. ) and thymol.

### (III-2) Esters

p-tert-Butylcyclohexyl acetate (Vertenex: manufactured by IFF), ethyl (2-methyl-[1,3] dioxolane-2-yl) acetate (Fraistone: manufactured by IFF), linalyl acetate, tricyclodecenyl propionate (Cyclaprop: manufactured by IFF), geranyl acetate, benzyl salicylate, hexyl salicylate, ethylene brassylate (MUSK T: manufactured by Takasago International Corp. ), methyl dihydroxydimethyl benzoate (Evernyl: manufactured by Givaudan), 3-pentyl tetrahydropyranyl acetate (Jasmal: manufactured by IFF), phenyl ethyl salicylate, ethyl 2-tert-butylcyclohexyl carboxylate (Floramat: manufactured by Henkel), 10-oxa-16-hexadecanolide (Oxalide: manufactured by Takasago International Corp. ), ethyl tricyclo [5.2.1.0^{2,6}] decan-2-yl carboxylate (Fruitate: manufactured by Kao Corp.), dimethylbenzylcarbinyl acetate and cedryl acetate.

### (III-3) Aldehydes

Dodecanal, 2-methyl-3-(4-isopropylphenyl)propanal (cyclamen aldehyde), α-hexyl cinnamic aldehyde, cumin aldehyde, 2,6,10-trimethyl-9-undecenal, 1-methyl-4-(4-methyl-3-pentenyl)-3-cyclohexen carboxy aldehyde (Homomyrac aldehyde : manufactured by IFF), nonanal, decanal and 3-dodecenal.

### (III-4) Ketones

β-Ionone, acetylcedrene (Woodyflor: manufactured by Takasago International Corp.), 7-acetyl-1,2,3,4,5,6,7,8- octahydro-1,1,6,7-tetramethyl naphthalene (Iso E Super: manufactured by IFF), 4- (4-hydroxyphenyl)-2-butanone (raspberry ketone), methyl naphthyl ketone and cis-jasmone.

### (III-5) Ethers

Hexamethyl hexahydrocyclopentabenzopyran (Glaxolide: manufactured by IFF) and cedryl methyl ether.

### (III-6) Nitrogen-containing compound

Indole.

Among these balance components, as compounds or essential oils excellent in harmonizing the fragrance to the sweat odor, in particular, to the armpit odor, there are illustrated;
(III-1) alcohols selected from dihydromyrcenol and isocamphyl cyclohexanol (Santalex: manufactured by Takasago International Corp. );
(III-2) esters selected from p-tert-butylcyclohexyl acetate (Vertenex: manufactured by IFF), ethyl (2-methyl- [1,3] dioxolane-2-yl) acetate (Frayston: manufactured by IFF), linalyl acetate, tricyclodecenyl propionate (Cyclaprop: manufactured by IFF), geranyl acetate, benzyl salicylate, hexyl salicylate, ethylene brassylate (MUSK T: manufactured by Takasago International Corp. ), methyl dihydroxydimethyl benzoate (Evernyl : manufactured by Givaudan) and cedryl acetate ;
(III-3) aldehydes selected from dodecanal, 2-methyl-3-(4-isopropylphenyl) propanal (cyclamen aldehyde) and α-hexyl cinnamic aldehyde;
(III-4) ketones selected from β-ionone, acetylcedrene (Woodyflor: manufactured by Takasago International Corp.), 7-acetyl-1,2,3,4,5,6,7,8-octahydro-1,1,6,7-tetramethyl naphthalene (Iso E Super: manufactured by IFF) and 4- (4-hydroxyphenyl)-2-butanone (raspberry ketone) ; and
(III-5) ethers selected from hexamethyl hydrocyclopentabenzopyran (Glaxolide: manufactured by IFF).

Each of the compounds or essential oils of the group (III) may be compounded singly or in combinations of two or more kinds thereof as the component (III) (balance component) in the compositions for inhibiting a body odor of the invention. A compounding amount of each of these balance components in the compositions for inhibiting a body odor of the invention is appropriately selected in accordance with components being specifically selected, the masking effect to be invested for masking the sweat odor, the armpit odor and so on, and further other compounding components; that is, it is not restricted to particular ones. In the present invention, by compounding the balance component, the masking effect can be excellently exhibited for the armpit odor. Thereby, the compositions for inhibiting a body odor of the invention that are useful for the deodorants, external preparations and so on including perfumeries can be provided.

As mentioned above, a compounding amount of each of the components (I), components (II) and components (III) is appropriately selected in accordance with components being specifically selected, the masking effect to be invested for masking the sweat odor, the armpit odor and so on, and further other compounding components; that is, it is not restricted to particular ones. However, as to the compounding amount thereof, the component (I) is generally used in the range of about from 40 to 95% by weight and more preferably in the range of from 50 to 85% by weight to a total amount of components (I) to (III). Further, a total amount of a mixture of perfume materials of components (I) and (II) is generally 60% by weight or more, and preferably in the range of from 60 to 100% by weight. Furthermore, the amount of component (III) is less than 50% by weight and preferably in the range of from 0 to 40% by weight. The reasons for this is in that when a total amount of the mixture of fragrance materials of components (I) and (II) is less than 60% by weight, usually, a sufficient masking effect against the acid odor cannot be obtained.

In the present invention, the selection of the above component (I), component (II) and component (III) was carried out as follows. That is, one of the pseudo body odor compositions mentioned above and a target fragrance component were mixed and left at room temperature for 30 minutes. Thereafter, evaluations of the masking and harmonizing effects to the acid odor were carried out by four expert panelists. In the present invention, by use of newly developed pseudo body odor compositions, the evaluations can be carried out efficiently. Specific evaluation methods, evaluation criteria and evaluation results will be explained in Experimental Example 1 described later.

### [Selection of components (I), (II) and (III)]

Results of evaluations of the masking effect and the harmonizing effect to the acid odor were put together and evaluated synthetically as follows, and thereby the components (I), (II) and (III) were selected as follows.
Component (I): Compounds or essential oils that exhibit 2 points or more of masking point in Experimental Example 1 described later in which the degree of masking due to lilial is set at 1 point, and 1 point of the harmonizing point in the harmonizing evaluation.
Component (II): Compounds or essential oils that exhibit 1 point or more of the masking point in the above masking evaluation in which the degree of masking due to lilial is set at 1 point, and 0 point of the harmonizing point in the harmonizing evaluation.
Component (III): Compounds or essential oils that exhibit 2 point or less of the masking point in the above masking evaluation in which the degree of masking due to lilial is set at 1 point, and -1 point of the harmonizing point in the harmonizing evaluation.

In the perfume compositions for inhibiting a body odor of the invention, other than the above fragrance components, perfume materials described in "Synthetic perfume, Chemical and Product Information, 1996/3/6, Kagaku Kogyo Nippousha and so on can be added to an extent that does not disturb a target effect of the invention, that is, within a quantitative and qualitative range that can eliminate the unpleasant sensation accompanying the acid odor. Other than the above, a solvent, a fixing agent and so on may be compounded.

The perfume compositions for inhibiting a body odor of the invention are excellent in the masking capability and/or harmonizing effect to the acid odor in the body odor and when these are added to, for instance, deodorant compositions or external preparations, the acid odor due to the body odor in particular the armpit odor can be effectively inhibited.

### (3) Deodorant compositions and external preparation compositions

The perfume compositions for inhibiting a body odor of the invention are compounded into, for instance, deodorant compositions or external preparation compositions in order to eliminate the unpleasant sensation accompanying the acid odor from the sweat and so on. To the deodorant compositions or external preparation compositions with which the perfume compositions for inhibiting a body odor of the invention are compounded, known additives and so on may be compounded within a range that does not disturb the target effect of the invention, that is, within a quantitative and qualitative range that can eliminate the unpleasant sensation accompanying the acid odor. As the additives other than the perfume compositions for inhibiting a body odor of the invention, there are illustrated, for example, an anti-oxidant, an antibacterial agent, a UV light absorber, a moisturizing agent, and drug components such as an anti-inflammatory agent, a skin whitening agent, an astringent agent, a refrigerant and a herbal medicine extract.

As the antioxidants, there are illustrated, for example, carotenoids such as α-carotin, β-carotin, γ-carotin, lycopene, cryptoxanthin, zeaxanthin, isozeaxanthin, rhodoxanthin, capsanthin, crocetin and so on; furans such as 2,5-dimethyl furan, 2-methyl furan, 2,5- diphenyl furan, 1, 3-diphenylisobenzofuran and so on; vitamin E's such as α-tocopherol, β-tocopherol, γ-tocopherol, δ-tocopherol and so on ; amino acids such as histidine, tryptophane, methionine, alanine, alkyl esters of alanine and so on; 1, 4-diadicyclooctane, dibutylhydroxy toluene (BHT), esters of gallic acids, ascorbic acid, tannic acids, flavonoids and so on; however, the antioxidant is not restricted thereto. The antioxidants each can be usually manufactured according to known methods, and it is not without saying that the commercially available products can be used.

Further, the extracts of natural medicines that have the antioxidizing effect can be used as the above antioxidizing agent. Specifically, extracts of the natural medicines such as an scutellaria extract, an Artemisia capillaris extract, a gambir extract, a sage extract, a tea extract, a rosemary extract, a fennel extract, a thyme extract, a nutmeg extract, a pepper extract, a turmeric extract, a vanilla extract, a paprika extract, a coix extract, a bupleurum radix extract, a chaenomeles extract, a caesalpinia sappan extract, a culen extract, Jungrahab (Baeckea frutescens L.) extract and so on can be cited but the extractions of the natural medicines are not restricted to the above.

When these antioxidizing agents are compounded into deodorant compositions, external preparation compositions and so on, an compounding amount thereof is roughly 0.0001 % by weight or more and 10.0% by weight or less and preferably roughly 0.01 % by weight or more and 1.0% by weight or less of the composition.

As the antibacterial agents, there are illustrated, for example, trichlorocarbanilide, benzalkonium chloride, benzethonium chloride, halocarbane, chlorohexidine hydrochloride, dihydrofarnesol (see, for example, JP-A No. 08-245979) and so on can be cited. When these antibacterial agents are compounded into deodorant compositions or external preparation compositions into which the perfume composition for inhibiting a body odor of the invention was compounded, an addition amount thereof is roughly 0.0001 % by weight or more and 10.0% by weight or less and preferably roughly 0.005% by weight or more and 5.0% by weight or less of the composition.

The dihydrofarnesol is known as an "antibacterial perfume" that combines functions of an antibacterial agent and a perfume. However, other perfumes than the dihydrofarnesol also can be compounded into the deodorant compositions or the external preparations compositions of the invention in order to mask or harmonize the acid odor in the event that it leaks.

For instance, in the case of the deodorant compositions and the external preparation compositions of the invention being used as a solvent composition, there can be compounded para-aminobenzoic acid base UV absorbers such as para-aminobenzoic acid and so on ; anthranilic acid base UV absorbers such as methyl anthranilate and so on; salicylic acid base UV absorbers such as octyl salicylate, phenyl salicylate, homomenthyl salicylate and so on; cinnamic acid base UV absorbers such as isopropyl para-methoxycinnamate, octyl para-methoxycinnamate, 2-ethylhexyl-paramethoxycinnamate, glyceryl mono-ethylhexanoyl-diparamethoxycinnamate, [4-bis (trimethylsiloxy) methylsilyl-3-methylbutyl] - 3,4,5- trimethoxy cinnamic acid ester and so on ; benzophenone base UV absorbers such as 2,4-dihydroxybenzophenone, 2-hydroxy- 4-methoxybenzophenone, 2-hydroxy-4-methoxybenzophenone-5-sulfonic acid, 2-hydroxy-4-methoxybenzophenone-5-sodium sulfonate and so on ; and UV absorbers such as urocanic acid, ethyl urocanate, 2-phenyl-5-methylbenzoxazole, 2- (2'- hydroxy-5'-methylphenyl)benzotriazole, 4-tert-butyl-4'- methoxydibenzoylmethane and so on.

Further, the moisturizing agents such as polyethylene glycol, propylene glycol, dipropylene glycol, 1, 3-butylene glycol, hexylene glycol, glycerin, diglycerin, xylitol, maltitol, maltose, D-mannite, starch syrup, glucose, fructose, lactose, chondroitin sodium sulfate, sodium hyaluronate, sodium adenosine phosphate, sodium lactate, bile acid salt, pyrrolidone carboxylic acid, glucosamine, cyclodextrin and so on also can be compounded into the deodorant compositions or external preparation compositions of the invention.

Furthermore, as the medicine components, there can also be compounded into the deodorant compositions or external preparation compositions of the invention, medicines having other medicinal virtues than the above anti-oxidizing agents such as vitamins such as inositol, pyridoxine hydrochloride, nicotinic acid benzyl, nicotinic acid amide, vitamin D₂ (ergocalciferol), biotin and so on; hormones such as estradiol, ethinyl estradiol and so on; antiinflammatory agents such as allantoin, azulene, glycyrrhetinic acid and so on; the skin whitening agents such as arbutin and so on; the astringents such as zinc oxide, tannic acid and so on; the refrigerants such as 1-Menthol, camphor and so on ; sulfur, lysozyme chloride and so on. In addition, the herbal extracts other than the herbal extracts that can be used as the above antioxidizing agent can be also compounded.

Other components capable of compounding into the deodorant compositions and the external preparation compositions of the invention are not restricted to the above ones. Further, each of the above illustrated drug efficacy components may be compounded singly into the external preparation compositions and may be compounded in appropriate combinations of two kinds or more thereof in accordance with the object. The other components may be also compounded into the deodorant perfume compositions of the invention, if necessary.

In the deodorant compositions and the external preparation compositions of the invention, known basis components and so on may be usually used within an extent that does not disturb the target effect of the invention according to desired modes and dosage forms thereof. That is, as the basis component, oil contents such as liquid oils and fats, liquid or solid oils and fats, solid oils and fats, waxes and so on can be compounded into skin care external preparation compositions. Further, ester oils, hydrocarbon oils and silicone may be compounded into the deodorant compositions and skin care external preparation compositions. Furthermore, surfactants such as anionic surfactants, cationic surfactants, and amphoteric surfactants, lipophilic non-ionic surfactants, hydrophilic non-ionic surfactants and so on can be compounded as the basis component.

Further, lower alcohols, sterols, plant polymers, microbial polymers, starch base polymers, animal base polymer, cellulose base polymers, alginic acid base polymers, vinyl base polymers, acrylic polymers, and watersoluble polymers may be compounded into the skin care external preparation compositions as the basis component, and metal ion sequestrants, neutralizers, pH adjusting agents and so on may be compounded as the basis component of the deodorant compositions and the external preparation compositions.

The basis components and so on shown here are only examples, that is, the basis components that can be compounded into the deodorant compositions and the external preparation compositions of the invention are not restricted to these basis components. The basis components can be appropriately combined and compounded into the deodorant compositions or the external preparation compositions in accordance with prescriptions corresponding to desired forms. Specific prescriptions of the deodorant compositions and the external preparation compositions will be described in Examples below.

Further, the perfume compositions for inhibiting a body odor of the invention can be used as means for inhibiting the acid odor that is liable to adhere to clothing and so on from occurring in the clothing and means for inhibiting the acid odor generated by human bodies from adhering to the clothing. Specifically, these can be used in the form of, for instance, a wash finishing agent of the clothing, a coating agent of the clothing and so on. In the case of the compositions being used in such forms, a fixing agent or the like for fixing, for instance, the perfume composition for inhibiting a body odor of the invention on a target object such as the clothing can be compounded into the compositions in accordance with the object. In the case of the compositions being used as the wash finishing agent of the clothing, the coating agent for the clothing and so on, the compositions are preferably used in the forms of, for instance, a liquid agent and a spray agent.

Furthermore, the perfume compositions for inhibiting a body odor of the invention can be put in prescriptions for preparing perfumeries and can be prepared into desired modes and dosage forms. As the modes and the dosage forms of thus prepared perfumeries, there are illustrated a perfume, an eau de cologne, an eau de toilette, a deodorant, soap, body soap, a shampoo, a detergent and so on as examples. In these cases, a compounding amount of the perfume composition according to the invention in the perfumery is not particularly restricted but is preferably in the range of about from 0.001 to 20.0% by weight relative to the whole amount of the perfumery.

The perfume compositions for inhibiting a body odor of the invention are compounded into various kinds of deodorants and the external preparation compositions, and exhibit excellent masking effect and/or harmonizing effect for the acid odors selected from hydroxyalkynyl carboxylic acids having 5 to 8 carbon atoms and alkenyl carboxylic acids having 5 to 8 carbon atoms and one double bond that are emitted from the sweat odor, in particular, the armpit odor. The deodorants and the external preparations wherein the perfume compositions for inhibiting a body odor of the invention are compounded may have the so-called deodorant effect as one of objects thereof. That is, the perfume compositions for inhibiting a body odor of the invention include all of so-called deodorants, external preparations such as skin external preparations and so on, wash finishing agents or coating agents for clothing as means for preventing emission of the acid odors that are liable to adhere to the clothing and so on or means for preventing adhesion of the acid odors emitted from human bodies to the clothing, perfumeries, and so on. That is, the deodorants and the external preparation compositions of the invention are restricted to the above examples, and denote all agents in which the perfume compositions for inhibiting a body odor of the invention are compounded to mask and/or harmonize effectively the acid odors emitted from the sweat odor, in particular, the armpit odor.

Thus, the deodorant compositions and the external preparation compositions of the invention are excellent in the masking and/or harmonizing effect to the acid odor in the body odors ; accordingly, the acid odors due to the body odor, in particular, the armpit odor can be effectively suppressed by use thereof.

### Examples

In the following, the present invention will be more specifically explained by reference to Examples. However, the present invention is not restricted to the following Examples and Comparative Examples.

### Examples 1 to 7 and Comparative Example 1

Pseudo sweat odor compositions for use in evaluation which have constitutions shown in Tables 2 and 3 of EP 1601338 A1 were prepared according to a standard method.

### Example 8

Using compounds of Table 4 of EP 1601338 A1 and a perfume composition A, suppression tests to evaluate the masking effect and the harmonizing effect of the body odor (sweat odor and armpit odor) were carried out according to a procedure of the following Experimental Example 1, test subjects were evaluated according to the following evaluation criteria, and the evaluations were shown as evaluated values. Results thereof are together shown in Table 4. In addition, a constitution of the perfume composition A in Table 4 is shown in Table 5.

### Experimental Example 1

Both a 2 cm x 2 cm filter paper with 200 µl of a pseudo body odor composition obtained according to Example 4 of EP 1601338 A1 and having a constituent shown in Table 6 below and a 2 cm x 2 cm filter paper with 10 µl of a perfume were put into a cup having an internal volume of about 500 ml, followed by sealing with an aluminum foil. After 30 minutes, a hole of about 1 cm in diameter was formed in the aluminum foil and the investigations of masking effect and the harmonizing effect were carried out according to following evaluation criteria by four expert panelists.

### [Evaluation criteria of the masking effect]

3: Excellent in the masking.
2: Substantially excellent in the masking.
1: Slightly sensible of the body odor (sweat odor and armpit odor).
0: Sensible of the body odor (sweat odor and armpit odor) (insufficient in the masking)

### [Evaluation criteria of the harmonizing effect]

1: In harmony.
0: Indefinite.
-1 : Not in harmony.

**Table 4**

| | Masking | Harmonizing |
|---|---|---|
| 2-Acetyl-2,3,8,8-tetramethyl octarin | 1 | -1 |
| β-Damascone | 2 | 1 |
| γ-Methylionone | 2 | 1 |
| Ethyl linalool | 1.5 | 0 |
| Eugenol | 1.5 | 0 |
| Coumarin | 2.5 | 1 |
| Geraniol | 2 | 1 |
| Phenylethyl acetate | 2.5 | 1 |
| cis-3-Hexenyl salicylate | 2 | 1 |
| cis-Jasmone | 1 | -1 |
| Citronellol | 2.5 | 1 |
| Phenylethyl alcohol | 2.5 | 1 |
| Phenyl ethyl dimethyl carbinol | 1.5 | -1 |
| Heliotropine | 1.5 | 0 |
| Benzaldehyde | 3 | 1 |
| Raspberry ketone | 0.5 | -1 |
| Linalool | 1 | 0 |
| Lemon oil | 3 | 1 |
| Rose oil | 2.5 | 1 |
| Rose oxide | 3 | 1 |
| Perfume composition A | 2.5 | 1 |

**Table 5**

| Constitution | Parts by weight |
|---|---|
| 2-Acetyl-2,3,8,8-tetramethyl octarin | 200 |
| γ-Methylionone | 5 |
| Methyl anthranilate | 4 |
| Coumarin | 5 |
| Geraniol | 80 |
| Trichloromethylphenylcarbinyl acetate | 20 |
| Phenylethyl acetate | 100 |
| cis-3-Hexenyl salicylate | 20 |
| cis-Jasmone | 1 |
| Citronellol | 50 |
| Farnesol | 50 |
| Phenylethyl alcohol | 90 |
| Phenyl ethyl dimethyl carbinol | 180 |
| Heliotropine | 100 |
| Linalool | 50 |
| Sandalwood oil | 15 |
| Bergamot oil | 10 |
| Thyme oil | 10 |
| Lemon oil | 10 |
| Total | 1000 |

### Example 9

According to the procedure of the Experimental Example 1 of EP 1601338A1, the suppression tests were conducted in the same manner as in Example 8 to evaluate the masking effect and the harmonizing effect of body odors (sweat odor and armpit odor) of many test subjects. On the basis of the results thereof, components (I) to (III) of the perfume compositions for inhibiting a body odor of the invention were selected. Results of the selections are shown in the following Tables 7-1 to 7-4 (I group), Tables 8-1 through 8-3 (II group), and Tables 9-1 through 9-3 (III group).

**Table 7-1**

| I group | Masking | Harmonizing |
|---|---|---|
| cis-3-Hexenol | 3 | 1 |
| cis-3-Hexenyl acetate | 3 | 1 |
| Tripral | 3 | 1 |
| Styralyl acetate | 3 | 1 |
| Allyl amyl glycolate | 3 | 1 |
| Citronellal | 3 | 1 |
| Citrathal | 3 | 1 |
| Hexanal | 3 | 1 |
| Octanal | 3 | 1 |
| Benzaldehyde | 3 | 1 |
| 2,6-Dimethyl-5-heptenal | 3 | 1 |
| Vigorose | 3 | 1 |
| Camphor | 3 | 1 |
| Methyl benzoate | 3 | 1 |
| p-Cresyl methyl ether | 3 | 1 |
| Citral | 3 | 1 |
| Geranyl nitrile | 3 | 1 |
| Citronellyl nitrile | 3 | 1 |
| Methyl heptenone | 3 | 1 |
| Ethyl acetoacetate | 3 | 1 |
| Allyl caproate | 3 | 1 |

**Table 7-2**

| I group | Masking | Harmonizing |
|---|---|---|
| Ethyl-2-methyl butylate | 3 | 1 |
| Ethyl-2-methyl valerate | 3 | 1 |
| α-Dynascone | 3 | 1 |
| Phenyl acetoaldehyde | 3 | 1 |
| Mentone | 3 | 1 |
| 1-Carvone | 3 | 1 |
| L-Rose oxide | 3 | 1 |
| Phenylethyl alcohol | 2.5 | 1 |
| Phenylethyl acetate | 2.5 | 1 |
| Citronellol | 2.5 | 1 |
| Benzyl acetate | 2.5 | 1 |
| Levosandol | 2.5 | 1 |
| Sandalmysore core | 2.5 | 1 |
| γ-Undecalactone | 2.5 | 1 |
| Cumarin | 2.5 | 1 |
| Ethyl vanillin | 2.5 | 1 |
| Borneol | 2.5 | 1 |
| Koavone | 2.5 | 1 |
| Methyl phenyl acetate | 2.5 | 1 |
| Isocyclocitral | 2.5 | 1 |
| Cyclogalbanate | 2.5 | 1 |

**Table 7-3**

| I group | Masking | Harmonizing |
|---|---|---|
| Acetoaldehydephenylethylpropyl acetal | 2.5 | 1 |
| Methyl anthranilate | 2.5 | 1 |
| 4-Methoxybenzaldehyde | 2.5 | 1 |
| γ-Nonalactone | 2.5 | 1 |
| γ-Decalactone | 2.5 | 1 |
| Geraniol | 2 | 1 |
| Damascenone | 2 | 1 |
| Cis-3-Hexenyl salicylate | 2 | 1 |
| Methylionone soap | 2 | 1 |
| Ambroxan | 2 | 1 |
| 2-tert-Butylcyclohexyl acetate | 2 | 1 |
| β-Damascone | 2 | 1 |
| Methyl dihydrojasmonate | 2 | 1 |
| Methylionone | 2 | 1 |
| Isobutyl quinoline | 2 | 1 |
| Phenylactoaldehyde dimethylacetal | 2 | 1 |
| Aurantiol | 2 | 1 |
| β-Naphthol methyl ether | 2 | 1 |
| Lemon oil | 3 | 1 |
| Thyme oil | 3 | 1 |
| Orange oil | 3 | 1 |

**Table 7-4**

| I group | Masking | Harmonizing |
|---|---|---|
| Rosemary oil | 3 | 1 |
| Ylang-ylang oil | 3 | 1 |
| Mandarin oil | 3 | 1 |
| Chamomile oil | 3 | 1 |
| Cardamom oil | 3 | 1 |
| Citronella oil | 3 | 1 |
| Elemi oil | 3 | 1 |
| Armoise oil | 3 | 1 |
| Galbanum oil | 3 | 1 |
| Peppermint oil | 3 | 1 |
| Eucalyptus oil | 2.5 | 1 |
| Lavandin oil | 2.5 | 1 |
| Cinammon oil | 2.5 | 1 |
| Petitgrain oil | 2.5 | 1 |
| Clary sage oil | 2.5 | 1 |
| Clove bud oil | 2.5 | 1 |
| Rose oil | 2.5 | 1 |
| Basil oil | 2.5 | 1 |
| Geranium oil | 2 | 1 |
| Taget oil | 2 | 1 |
| Spearmint oil | 2 | 1 |

**Table 8-1**

| II group | Masking | Harmonizing |
|---|---|---|
| Allylionone | 2.5 | 0 |
| 4-Methyl-3-decene-5-ol | 2.5 | 0 |
| Allyl cyclohexyl propionate | 2 | 0 |
| Terpinyl acetate | 2 | 0 |
| α-Damascone | 2 | 0 |
| Tetrahydromyrcenol | 2 | 0 |
| 4-tert-Butyl-2,6-dimethylacetophenone | 2 | 0 |
| Phenylethyl phenyl acetate | 2 | 0 |
| Ethyl-3-methyl-3-phenyl glycidate | 2 | 0 |
| Cinnamic aldehyde | 2 | 0 |
| Thesaron | 2 | 0 |
| Ethyl linalool | 1.5 | 0 |
| Tricyclodecenyl acetate | 1.5 | 0 |
| Citronellyl acetate | 1.5 | 0 |
| δ-Damascone | 1.5 | 0 |
| Eugenol | 1.5 | 0 |
| Heliotropine | 1.5 | 0 |
| Tonalid | 1.5 | 0 |
| Tridecene-2-nitrile | 1.5 | 0 |

**Table 8-2**

| II group | Masking | Harmonizing |
|---|---|---|
| 9-Decen-1-ol | 1.5 | 0 |
| 2-Methyl undecanal | 1.5 | 0 |
| 4-Tricyclodecylene butanal | 1.5 | 0 |
| p-tert-Butylcyclohexanol | 1.5 | 0 |
| Dimethyl octanol | 1.5 | 0 |
| Diphenyl oxide | 1.5 | 0 |
| Rosephenone | 1.5 | 0 |
| Floralozone | 1.5 | 0 |
| Dihydroroseoxide | 1.5 | 0 |
| Myracaldehyde | 1.5 | 0 |
| Anisyl acetate | 1.5 | 0 |
| Ambretone | 1.5 | 0 |
| Lilial | 1 | 0 |
| Kovanol | 1 | 0 |
| Hydroxycitronellal | 1 | 0 |
| Helional | 1 | 0 |
| Amyl salicylate | 1 | 0 |
| Floropal | 1 | 0 |

**Table 8-3**

| III group | Masking | Harmonizing |
|---|---|---|
| Dihydrojasmone | 1 | 0 |
| Cedar wood oil | 2 | 0 |
| Juniperberry oil | 2 | 0 |
| Thyme oil | 2 | 0 |
| Clove leaf oil | 1.5 | 0 |
| Nutmeg oil | 1.5 | 0 |

**Table 9-1**

| III group | Masking | Harmonizing |
|---|---|---|
| Dodecanal | 1.5 | -1 |
| Dihydromyrcenol | 1.5 | -1 |
| p-tert-Butylcyclohexyl acetate | 1.5 | -1 |
| Cyclamen aldehyde | 1.5 | -1 |
| β-Ionone | 1.5 | -1 |
| Woodyflor | 1.5 | -1 |
| Dimethylphenylethylcarbinol | 1.5 | -1 |
| Cumin aldehyde | 1.5 | -1 |
| Fluitate | 1.5 | -1 |
| Methyl naphthyl ketone | 1.5 | -1 |
| Indole | 1.5 | -1 |
| Nonanal | 1.5 | -1 |
| Fraistone | 1 | -1 |
| Linalyl acetate | 1 | -1 |
| Cyclaprop | 1 | -1 |

**Table 9-2**

| III group | Masking | Harmonizing |
|---|---|---|
| Geranyl acetate | 1 | -1 |
| α-Hexyl cinnamic aldehyde | 1 | -1 |
| Benzyl salicylate | 1 | -1 |
| Hexyl salicylate | 1 | -1 |
| Iso E super | 1 | -1 |
| 2,6,10-Trimethyl-9-undecenal | 1 | -1 |
| Homomyracaldehyde | 1 | -1 |
| 2-Phenyl propanol | 1 | -1 |
| Jasmal | 1 | -1 |
| Phenylethyl salicylate | 1 | -1 |
| Thymol | 1 | -1 |
| Decanal | 1 | -1 |
| 1-Menthol | 1 | -1 |
| Dimethylbenzyl carbinyl acetate | 1 | -1 |
| cis-Jasmone | 1 | -1 |
| Myol | 1 | -1 |
| Cedryl methyl ether | 1 | -1 |
| Santalex | 0.5 | -1 |
| Raspberry ketone | 0.5 | -1 |

**Table 9-3**

| III group | Masking | Harmonizing |
|---|---|---|
| Galaxolide | 0.5 | -1 |
| Musk T | 0.5 | -1 |
| Floramat | 0.5 | -1 |
| 2, 2-Dimethyl-3-(3-methylphenyl)propanol | 0.5 | -1 |
| 3-Dodecenal | 0.5 | -1 |
| α,α-Dimethylphenylethyl | 0.5 | -1 |
| Amber core | 0.5 | -1 |
| Evernyl | 0 | -1 |
| Oxalide | 0 | -1 |
| Cedryl acetate | 0 | -1 |
| Ambestol | 0 | -1 |

### Example 10 and Comparative Example 2

According to prescriptions of the following Table 10 (10-1 and 10-2), perfume compositions for detergent of Example 10 and Comparative Example 2 were prepared according to the standard method, and the masking effect and the harmonizing effect thereof were evaluated according to the method of the Experimental Example 1. The prescriptions are based on parts by weight (the same in the prescriptions of the following perfume compositions).

**Table 10-1 (perfume compositions for detergent)**

| | Example 10 | Comparative Example 2 |
|---|---|---|
| Lemon oil | 250 | |
| Thyme oil | 100 | |
| Citronellyl nitrile | 70 | 70 |
| Citronella oil | 1 | 1 |
| Octanal | 8 | 8 |
| 2-Methyl undecanal | 24 | |
| Dodecanal | | 24 |
| cis-3-Hexenol | 5 | 5 |
| Tripral | 40 | 40 |
| Eucalyptus oil | 20 | 20 |
| Tetrahydromyrcenol | 100 | |
| Dihydromyrcenol | | 450 |
| Lavandin oil | 100 | |
| Linalool | | 40 |
| Linalyl acetate | | 60 |
| 2-tert-Butylcyclohexyl acetate | 20 | |
| p-tert-Butylcyclohexyl acetate | | 20 |
| Tricyclodecenyl acetate | 20 | |
| Cyclaprop | | 20 |
| Citronellol | 30 | 30 |

**Table 10-2 continuation of Table 10-1)**

| | | |
|---|---|---|
| L-Rose oxide | 5 | 5 |
| Lilial | 60 | |
| α-Hexyl cinnamic aldehyde | | 60 |
| Floralozone | 2 | 2 |
| Koavone | 30 | |
| Woodyflor | | 30 |
| Methylionone soap | 30 | |
| β-Ionone | | 30 |
| 4-Methoxybenzaldehyde | 10 | |
| Heliotropine | | 10 |
| Dipropylene glycol | 75 | 75 |
| Total | 1000 | 1000 |

The perfume composition for detergent of Example 10 includes the component (I) by 719/1000, the component (II) by 206/1000, the component (III) by 0/1000 and other component by 75/1000. On the other hand, the perfume composition of Comparative Example 2 includes the component (I) by 179/1000, the component (II) by 12/1000, the component (III) by 694/1000 and other component by 115/1000.

An evaluated value of the masking effect of the perfume composition for detergent of Example 10 was 3.0 and that of the harmonizing effect was 1.0. From the evaluated value, it was found that the perfume composition of Example 10 is excellent in the masking effect and the harmonizing effect to the acid odor.

On the contrary, an evaluated value of the masking effect of the perfume composition for detergent according to Comparative Example 2 was 1.0 and that of the harmonizing effect was 0.0. From the evaluated value, both of the masking effect and the harmonizing effect of the perfume composition for detergent according to Comparative Example 2 were not satisfying.

In addition, the perfume composition for detergent of the invention was compounded according to the following prescription to prepare a detergent for clothing, which was a deodorant composition of the invention. This detergent showed excellent acid odor inhibiting effect due to the masking action and the harmonizing action as follows.

### Example 11 and Comparative Example 3

The perfume compositions for detergent of Example 10 and Comparative Example 2 were compounded according to the following prescription example of detergent for clothing to prepare detergents for clothing.

### [Prescription example of detergent for clothing]

| Component | % by weight |
|---|---|
| Sodium linear dodecylbenzene sulfonate | 10 |
| α-sulfo fatty acid methyl ester sodium salt | 10 |
| Dodecanol EO (25) adduct | 2 |
| C12 to C13 fatty acid sodium salt | 5 |
| Zeolite | 20 |
| Sodium silicate | 7 |
| Sodium carbonate | 17 |
| Polyethylene glycol | 1 |
| Carboxy methyl cellulose | 1 |
| Perfume composition | 5 |
| Sodium sulfate | balance |
| Total | 100 |

Washing was carried out by use of the detergents of Example 11 and Comparative Example 3 under the following experimental conditions, and the odor intensities of the acid odors in laundry due to the armpit odor were compared and evaluated according to evaluation criteria below by five expert panelists. Results are shown in Table 11 below. As obvious from Table 11, the laundry washed by use of the detergent for clothing of Example 10 showed excellent acid odor inhibiting effect due to the masking action and the harmonizing action of the perfume composition for detergent according to Example 9.

On the contrary, the laundry washed by use of the detergent for clothing according to Comparative Example 3 showed insufficient acid odor inhibiting effect and the acid odor therein was detected even after the washing.

### [Experimental conditions]

After taking a bath, an examinee having strong armpit odor was got to wear a shirt with previously washed gauzes attached on the places contacting with armpits directly. After 24 hours, the gauzes were washed and rinsed followed by dewatering. An odor just after the dewatering was evaluated by five expert panelists.

### [Evaluation criteria]

○ : The acid odor is sufficiently inhibited.
△ : Slight acid odor is detected.
X: Obvious acid odor is detected.

### [Results of evaluation]

**Table 11**

| | Example 11 | Comparative Example 3 |
|---|---|---|
| Evaluation | ○ | △ |

### Example 12 and Comparative Example 4

According to prescriptions of the following Table 12 (12-1 and 12-2), perfume compositions for soap of Example 12 and Comparative Example 4 were prepared according to a standard method followed by evaluating the masking effect and the harmonizing effect thereof according to the method of Experimental Example 1.

**Table 12-1 (perfume composition for soap)**

| | Example 12 | Comparative Example 4 |
|---|---|---|
| 9-Decen-1-ol | 25 | |
| Dodecanal | | 25 |
| Geranium oil | 10 | 10 |
| Tetrahydromyrcenol | 50 | |
| Dihydromyrcenol | | 50 |
| Terpinyl acetate | 50 | |
| Lavandin oil | 100 | |
| Linalool | | 40 |
| Linalyl acetate | | 110 |
| 2-tert-Butylcyclohexyl acetate | 100 | |
| p-tert-Butylcyclohexyl acetate | | 100 |
| Tricyclodecenyl acetate | 10 | |
| Cyclacrop | | 10 |
| p-tert-Butylcyclohexanol | 5 | 5 |
| Phenylethyl alcohol | 250 | |
| α-Hexyl cinnamic aldehyde | | 250 |
| Citronellal | 80 | |
| Jasmal | | 80 |
| β-Damascenone | 3 | 3 |
| L-Rose oxide | 1 | 1 |
| Benzyl acetate | 30 | 30 |

**Table 12-2 (continuation of Table 12-1)**

| | | |
|---|---|---|
| Lilial | 50 | |
| cis-p-Isopropylcyclohexyl methanol | | 50 |
| Methylionone soap | 75 | |
| Woodyflor | | 75 |
| Ambroxan | 1 | 1 |
| Bacdanol | 10 | |
| Santalex | | 10 |
| Heliotropine | | 50 |
| Coumarin | 50 | |
| Tonalid | 50 | |
| Glaxolide | | 100 |
| Dipropylene glycol | 50 | |
| Total | 1000 | 1000 |

The perfume composition for soap according to Example 12 includes the component (I) by 710/1000, the component (II) by 240/1000, the component (III) by 0/1000 and other component by 50/1000 ; on the other hand, the perfume composition according to Comparative Example 4 includes the component (I) by 45/1000, the component (II) by 55/1000, the component (III) by 860/1000 and other component by 40/1000.

An evaluated value of the masking effect of the perfume composition for soap according to Example 12 was 3.0 and that of the harmonizing effect was 1.0. From the evaluated value, it was found that the perfume composition of Example 12 is excellent in the masking effect and the harmonizing effect to the acid odor.

On the contrary, an evaluated value of the masking effect of the perfume composition for soap according to Comparative Example 4 was 1.0 and that of the harmonizing effect was 0.0, that is, both of the masking effect and the harmonizing effect of the perfume composition for soap according to Comparative Example 4 were not satisfying.

### Example 13 and Comparative Example 5

The perfume compositions for soap of Example 12 and Comparative Example 4 were compounded according to the following example of soap prescription to prepare soaps.

### [Example of soap prescription]

| Component | % by weight |
|---|---|
| Coconut/perm oil fatty acid sodium salt | 90 |
| Coconut/perm oil fatty acid | 3 |
| Dibutylhydroxytoluene | 0.1 |
| EDTA | 0.1 |
| Citric acid | 0.5 |
| Titanium oxide | 0.3 |
| Perfume composition | 1 |
| Purified water | balance |
| Total | 100 |

The soaps of Example 13 and Comparative Example 5 were used under the following conditions, and then the intensities of odors of the acid odors due to the armpit odor after the use were compared and evaluated by five expert panelists. Results are shown in Table 13 below. As obvious from Table 13, when the washing was carried out by use of the soap according to Example 13, excellent acid odor inhibiting effect due to the masking action and the harmonizing action of the perfume composition for soap according to Example 12 was found in the gauze. On the contrary, when the washing was carried out by use of the soap according to Comparative Example 5, the acid odor was detected in the gauze.

### [Experimental conditions]

After taking a bath, an examinee having strong armpit odor was got to wear a shirt with previously washed gauzes attached on the places contacting with armpits directly. After 24 hours, the gauzes were washed with the soap and rinsed followed by dewatering. An odor just after the dewatering was evaluated according to evaluation criteria below by five expert panelists.

### [Evaluation criteria]

○ : The acid odor is sufficiently inhibited.
△ : Slight acid odor is detected.
X: Obvious acid odor is detected.

### [Results of evaluation]

**Table 13**

| | Example 13 | Comparative Example 5 |
|---|---|---|
| Evaluation | ○ | △ |

### Example 14 and Comparative Example 6

According to prescriptions shown in the following Table 14 (14-1 and 14-2), perfume compositions for body soap of Example 14 and Comparative Example 6 were prepared according to a standard method followed by evaluating the masking effect and the harmonizing effect thereof according to the method of Experimental Example 1.

**Table 14-1 (perfume composition for body soap)**

| | Example 14 | Comparative Example 6 |
|---|---|---|
| Bergamot oil | 120 | |
| Linalool | | 50 |
| Linalyl acetate | | 70 |
| Lemon oil | 100 | |
| Thyme oil | 150 | |
| Dihydromyrcenol | | 250 |
| Citronellyl nitrile | 20 | |
| Tridecene-2-nitrile | | 20 |
| Octanal | 3 | |
| Decanal | | 3 |
| Allyl caproate | 10 | |
| Fruitate | | 10 |
| cis-3-Hexenol | 2 | 2 |
| Styralyl acetate | 20 | |
| Floropal | | 20 |
| Citronellol | 80 | |
| Jasmal | | 80 |
| Citronellyl acetate | 20 | |
| Benzyl acetate | 20 | |
| Dimethyl benzyl carbinyl acetate | | 40 |

**Table 14- 2 (continuation of Table 14-1)**

| | | |
|---|---|---|
| βDamascenone | 10 | 10 |
| Lilial | 80 | |
| cis-p-Isopropylcyclohexyl methanol | | 80 |
| Methyl dihydrojasmonate | 150 | |
| α-Hexyl cinnamic aldehyde | | 150 |
| Myracaldehyde | 15 | |
| 3-Dodecenal | | 15 |
| cis-3-Hexenyl salicylate | 20 | |
| Hexyl salicylate | | 20 |
| Methylionone soap | 50 | |
| Woodyflor | | 50 |
| Ambroxan | 10 | |
| Evernyl | | 10 |
| Sandalmysore core | 20 | |
| Santalex | | 20 |
| Tonalid | 50 | |
| Glaxolide | | 100 |
| Dipropylene glycol | 50 | |
| Total | 1000 | 1000 |

The perfume composition for body soap of Example 14 includes the component (I) by 665/1000, the component (II) by 165/1000, the component (III) by 0/1000 and other component by 170/1000. On the other hand, the perfume composition of Comparative Example 6 includes the component (I) by 12/1000, the component (II) by 40/1000, the component (III) by 898/1000 and other component by 50/1000.

An evaluated value of the masking effect of the perfume composition for body soap according to Example 14 was 3.0 and that of the harmonizing effect was 1.0. From the evaluated value, it was found that the perfume composition of Example 14 is excellent in the masking effect and the harmonizing effect to the acid odor.

On the contrary, an evaluated value of the masking effect of the perfume composition for body soap according to Comparative Example 6 was 1.0 and that of the harmonizing effect was 0.0, that is, both of the masking effect and the harmonizing effect of the perfume according to Comparative Example 6 were not satisfying.

### Example 15 and Comparative Example 7

The perfume compositions for body soap of Example 14 and Comparative Example 6 were compounded according to the following example of body soap prescription to prepare body soaps.

### [Example of body soap prescription]

| Component | % by weight |
|---|---|
| Potassium hydroxide | 3 |
| Lauric acid | 6 |
| Sodium lauryl polyoxyethylene sulfate | 10 |
| Pearlescent agent | 10 |
| Lauryl amide propyl betaine | 8 |
| Perfume composition | 0.5 |
| Purified water | Balance |
| Total | 100 |

The body soaps of Example 14 and Comparative Example 7 were used under the following conditions, and then the intensities of odors of the acid odors due to the armpit odor after the use were compared and evaluated according to evaluation criteria below by five expert panelists. Results are shown in Table 15 below. As obvious from Table 15, when the washing was carried out by use of the body soap of Example 15, excellent acid odor inhibiting effect due to the masking action and the harmonizing action of the perfume composition for body soap was found in the gauze. On the contrary, when the washing was carried out by use of the body soap according to Comparative Example 7, the acid odor was detected in the gauze.

### [Experimental conditions]

After taking a bath, an examinee having strong armpit odor was got to wear a shirt with previously washed gauzes attached on the places contacting with armpits directly.

After 24 hours, the gauzes were washed with the body soaps and rinsed followed by dewatering. An odor just after the dewatering was evaluated according to evaluation criteria below by five expert panelists.

### [Evaluation criteria]

○: The acid odor is sufficiently inhibited.
△: Slight acid odor is detected.
X: Obvious acid odor is detected.

### [Results of evaluation]

**Table 15**

| | Example15 | Comparative Example 7 |
|---|---|---|
| Evaluation | ○ | Δ |

### Example 16 and Comparative Example 8

According to prescriptions shown in the following Table 16 perfume compositions for deodorant of Example 16 and Comparative Example 8 were prepared according to a standard method followed by evaluating the masking effect and the harmonizing effect thereof according to the method of Experimental Example 1.

**Table 16 (perfume composition for deodorant)**

| | Example 16 | Comparative Example 8 |
|---|---|---|
| Bergamot oil | 200 | |
| Linalool | | 80 |
| Linalyl acetate | | 120 |
| Lemon oil | 150 | 150 |
| Petitgrain oil | 10 | 10 |
| cis-3-Hexenol | 3 | 3 |
| Tripral | 3 | 3 |
| Allyl amyl glycolate | 3 | 3 |
| Galbanum oil | 1 | 1 |
| Clary sage oil | 10 | 10 |
| Citronellol | 100 | 100 |
| Methyl dihydrojasmonate | 300 | |
| Jasmal | | 300 |
| β-Damscenone | 10 | 10 |
| Benzyl acetate | 20 | 20 |
| β-Naphthol methyl ether | 5 | 5 |
| Dihydrorose oxide | 80 | |
| cis-p-Isopropylcyclohexyl methanol | | 80 |
| cis-3-Hexenyl salicylate | 20 | |
| Hexyl salicylate | | 20 |
| Methylionone soap | 30 | |
| β-lonone | | 30 |
| γ-Undecalactone | 5 | 5 |
| Tonalid | 25 | |
| Glaxolide | | 50 |
| Dipropylene glycol | 25 | |
| Total | 1000 | 1000 |

The perfume composition for deodorant of Example 16 includes the component (I) by 670/1000, the component (II) by 105/1000, the component (III) by 0/1000 and other component by 225/1000. On the other hand, the perfume composition of Comparative Example 8 includes the component (I) by 320/1000, the component (II) by 0/1000, the component (III) by 600/1000 and other component by 80/1000.

An evaluated value of the masking effect of the perfume composition for deodorant according to Example 16 was 3.0 and that of the harmonizing effect was 1.0. From the evaluated value, it was found that the perfume composition of Example 16 is excellent in the masking effect and the harmonizing effect to the acid odor.

On the contrary, an evaluated value of the masking effect of the perfume composition for deodorant according to Comparative Example 8 was 1. 0 and that of the harmonizing effect was 0.0, that is, both of the masking effect and the harmonizing effect of perfume composition for deodorant according to Comparative Example 8 were not satisfying.

### Example 17 and Comparative Example 9

The perfume compositions for deodorant of Example 16 and Comparative Example 8 were compounded according to the following example of prescription to prepare lotions that are external preparation composition.

### [Example of deodorant (lotion) prescription]

| Component | % by weight |
|---|---|
| Aluminum chlorohydrate | 35 |
| Ethanol | 35 |
| Hydroxyethyl cellulose | 0.6 |
| Chlorhexidine gluconate | 0.2 |
| Polyoxyethylene polyoxypropylene cetyl ether | 2 |
| Perfume composition | 0.5 |
| Purified water | balance |
| Total | 100 |

The lotions of Example 17 and Comparative Example 9 were used under the following conditions, and then the intensities of odors of the acid odors due to the armpit odor after the use were compared and evaluated according to evaluation criteria below by five expert panelists. Results are shown in Table 17 below. As obvious from Table 17, in the treatment by use of the lotion according to Example 17, excellent acid odor inhibiting effect due to the masking action and the harmonizing action of the perfume composition for lotion was found in the gauze. On the contrary, in the treatment with the lotion of Comparative Example 9, the acid odor was detected in the gauze.

### [Experimental conditions]

After taking a bath, an examinee having strong armpit odor was got to treat one armpit with the deodorant agent and to wear a shirt with previously washed gauzes attached on the places contacting with armpits directly. After 24 hours, the odors of the gauzes were evaluated by five expert panelists.

### [Evaluation criteria]

○: Better than non-applied.
△: Slightly better than non-applied. (Slight acid odor is detected.)
X: Equivalent to non-applied. (Obvious acid odor is detected.)

### [Results of evaluation]

**Table 17**

| | Example17 | omparative Example 9 |
|---|---|---|
| Evaluation | O | △ |

### Example 18 and Comparative Example 10

According to prescriptions shown in the following Table 18 (18-1 and 18-2), perfume compositions for deodorant of Example 18 and Comparative Example 10 were prepared according to a standard method followed by evaluating the masking effect and the harmonizing effect thereof according to the method of Experimental Example 1 of EP 1601338A1.

**Table 18-1 (perfume composition for deodorant)**

| | Example 18 | Comparative Example 10 |
|---|---|---|
| Lemon oil | 60 | 60 |
| Mandarin oil | 40 | 40 |
| Bergamot oil | 250 | |
| Linalool | | 100 |
| Linalyl acetate | | 150 |
| Ethyl acetoacetate | 12 | 12 |
| Clary sage oil | 8 | 8 |
| β-Damascenone | 20 | 20 |
| Methyl dihydrojasmonate | 300 | |
| Jasmal | | 300 |
| Kovanol | 80 | |
| Myol | | 80 |
| Floralozone | 5 | |
| 2, 2-Dimethyl-3-(3-methylphenyl) propanol | | 5 |
| cis-3-Hexenyl salicylate | 50 | |
| Hexyl salicylate | | 50 |
| Nutmeg oil | 3 | |
| Clove leaf oil | | 3 |
| β-Ionone | 30 | |
| Iso E super | | 30 |
| Ambroxane | 2 | 2 |
| Sandalmysore core | 40 | |
| Santalex | | 40 |
| Tonalid | 50 | |
| Glaxolide | | 100 |
| Dipropylene glycol | 50 | |
| Total | 1000 | 1000 |

The perfume composition for deodorant of Example 18 includes the component (I) by 532/1000, the component (II) by 138/1000, the component (III) by 30/1000 and other component by 300/1000. On the other hand, the perfume composition of Comparative Example 9 includes the component (I) by 142/1000, the component (II) by 3/1000, the component (III) by 755/1000 and other component by 100/1000.

An evaluated value of the masking effect of the perfume composition for deodorant according to Example 18 was 3.0 and that of the harmonizing effect was 1.0. From the evaluated value, it was found that the perfume composition of Example 18 is excellent in the masking effect and the harmonizing effect to the acid odor.

On the contrary, an evaluated value of the masking effect of the perfume composition for deodorant according to Comparative Example 10 was 1.0 and that of the harmonizing effect was 0.0, that is, both of the masking effect and the harmonizing effect of perfume composition for deodorant according to Comparative Example 10 were not satisfying.

### Example 19 and Comparative Example 11

The perfume compositions for deodorant of Example 18 and Comparative Example 10 were compounded according to the following example of prescription to prepare spray-like deodorants (lotion type) that are external preparation composition.

### [Example of prescription for spray-like deodorant (lotion type)]

| Component | % by weight |
|---|---|
| Hydroxyethyl cellulose | 0.6 |
| Aluminum chlorohydroxide | 18 |
| Ethanol | 10 |
| Propylene glycol | 15 |
| Talc | 7 |
| Polyoxyethylene monodistearate | 3 |
| Perfume composition | 0.05 |
| Purified water | balance |
| Total | 100 |

The spray-like deodorants (lotion type) of Example 19 and Comparative Example 11 were used under the following conditions, and then the intensities of odors of the acid odors due to the armpit odor after the use were compared and evaluated according to evaluation criteria below by five expert panelists. Results are shown in Table 19 below. As obvious from Table 19, in the treatment by use of the spray-like deodorant according to Example 19, excellent acid odor inhibiting effect due to the masking action and the harmonizing action of the perfume composition for deodorant was found in the gauze. On the contrary, in the treatment with the spray-like deodorant (lotion type) of Comparative Example 11, the acid odor was detected in the gauze even after the use.

### [Experimental conditions]

After taking a bath, an examinee having strong armpit odor was got to treat one armpit with a deodorant agent and to wear a shirt with previously washed gauzes attached on the places contacting with armpits directly. After 24 hours, the odors of the gauzes were evaluated by five expert panelists.

### [Evaluation criteria]

○: Better than non-applied.
△: Slightly better than non-applied. (Slight acid odor is detected.)
X: Equivalent to non-applied. (Obvious acid odor is detected.)

### [Results of evaluation]

**Table 19**

| | Example 19 | Comparative Example 11 |
|---|---|---|
| Evaluation | ○ | △ |

### Example 20 and Comparative Example 12

According to an example of prescription shown below, stock solutions of perfume compositions for deodorant of Example 18 and Comparative Example 10 were prescribed, and thereby aerosols (for use in aerosol container) that are external preparation compositions were prepared.

### [Example of prescription for aerosol (for use in aerosol container)]

| Component | | % by weight |
|---|---|---|
| Above stock solution | | 45 |
| LPG | | 55 |
| Total | | 100 |

The aerosols of Example 20 and Comparative Example 12 were used under the following conditions, and then the intensities of odors of the acid odors due to the armpit odor after the use were compared and evaluated according to evaluation criteria below by five expert panelists. Results are shown in Table 20 below. As obvious from Table 20, in the treatment by use of the aerosol according to Example 20, excellent acid odor inhibiting effect due to the masking action and the harmonizing action of the perfume composition for deodorant according to Example 18 was found in the gauze. On the contrary, in the treatment with the lotion of Comparative Example 12, the acid odor was detected in the gauze even after the use.

### [Experimental conditions]

After taking a bath, an examinee having strong armpit odor was got to treat one armpit with the aerosol and to wear a shirt with previously washed gauzes attached on the places contacting with armpits directly. After 24 hours, the odors of the gauzes were evaluated by five expert panelists.

### [Evaluation criteria]

○: Better than non-applied.
△: Slightly better than non-applied. (Acid odor is slightly sensed.)
X: Equivalent to non-applied. (Acid odor is apparently sensed.)

### [Results of evaluation]

**Table 20**

| | Example 20 | Comparative Example 12 |
|---|---|---|
| Evaluation | ○ | △ |

### Example 21 and Comparative Example 13

According to prescriptions shown in the following Table 21, perfume compositions for aerosol powder of Example 21 and Comparative Example 13 were prepared according to a standard method followed by evaluating the masking effect and the harmonizing effect thereof according to the method of Experimental Example 1.

**Table 21 (perfume composition for aerosol powder)**

| | Example 21 | Comparative Example 13 |
|---|---|---|
| Ethyl-2-methyl butylate | 10 | 10 |
| Ethyl-2-methyl valerate | 50 | |
| Fraistone | | 50 |
| Allyl caproate | 80 | 80 |
| Allyl amyl glycolate | 30 | 30 |
| Tripral | 50 | 50 |
| Terpinyl acetate | 80 | |
| Linalyl acetate | | 80 |
| 2-tert-Butylcyclohexyl acetate | 400 | |
| p-tert-Butylcyclohexyl acetate | | 400 |
| Koavone | 150 | |
| α-Hexyl cinnamic aldehyde | | 150 |
| γ-Undecalactone | 30 | 30 |
| Coumarin | 20 | |
| Heliotropine | | 20 |
| Tonalid | 50 | |
| Glaxolide | | 100 |
| Dipropylene glycol | 50 | |
| Total | 1000 | 1000 |

The perfume composition for aerosol powder of Example 21 includes the component (I) by 820/1000, the component (II) by 130/1000, the component (III) by 0/1000 and other component by 50/1000. On the other hand, the perfume composition of Comparative Example 13 includes the component (I) by 200/1000, the component (II) by 20/1000, the component (III) by 780/1000 and other component by 0/1000.

An evaluated value of the masking effect of the perfume composition for aerosol powder according to Example 21 was 3.0 and that of the harmonizing effect was 1.0. From the evaluated value, it was found that the perfume composition of Example 21 is excellent in the masking effect and the harmonizing effect to the acid odor.

On the contrary, an evaluated value of the masking effect of the perfume composition for aerosol powder according to Comparative Example 13 was 1.0 and that of the harmonizing effect was 0.0 ; that is, both of the masking effect and the harmonizing effect of the perfume composition for aerosol powder according to Comparative Example 13 were not satisfying.

### Example 22 and Comparative Example 14

The perfume compositions for aerosol powder of Example 21 and Comparative Example 13 were compounded according to the following example of prescription to prepare aerosol powders that are deodorant composition.

### [Example of prescription for aerosol powder]

| Component | % by weight |
|---|---|
| Aluminum chloride hydroxide | 20 |
| Magnesia-silica | 15 |
| Silicic anhydride | 7 |
| POE (20) glycerol triisostearate | 15 |
| Methyl polysiloxane | 2 |
| Decamethyl cyclopentasiloxane | 1 |
| Perfume composition | 0.05 |
| Isopropyl myristate | balance |
| Total | 100 |

The aerosol powders of Example 22 and Comparative Example 14 were used under the following conditions, and then the intensities of odors of the acid odors due to the armpit odor after the use were compared and evaluated according to evaluation criteria below by five expert panelists. Results are shown in Table 22 below. As obvious from Table 22, in the treatment by use of the aerosol powder according to Example 22, excellent acid odor inhibiting effect due to the masking action and the harmonizing action of the perfume composition for deodorant according to Example 21 was found in the gauze. On the contrary, in the treatment with the aerosol powder of Comparative Example 14, the acid odor was detected in the gauze even after the use.

### [Experimental conditions]

After taking a bath, an examinee having strong armpit odor was got to treat one armpit with the aerosol and to wear a shirt with previously washed gauzes attached on the places contacting with armpits directly. After 24 hours, the odors of the gauzes were evaluated by five expert panelists.

### [Evaluation criteria]

○: Better than non-applied.
△: Slightly better than non-applied. (Acid odor is slightly sensed.)
X: Equivalent to non-applied. (Acid odor is apparently sensed.)

### [Results of evaluation]

**Table 22**

| | Example 22 | Comparative Example 14 |
|---|---|---|
| Evaluation | ○ | △ |

### Example 23 and Comparative Example 15

According to an example of prescription shown below, stock solutions of perfume compositions for aerosol powder according to Example 21 and Comparative Example 13 were prescribed to prepare aerosol powders (for aerosol container) that are deodorant compositions.

### [Example of prescription for powder aerosol (for aerosol container)]

| Component | | % by weight |
|---|---|---|
| Above stock solution | | 45 |
| LPG | | 55 |
| Total | | 100 |

The aerosol powders of Example 23 and Comparative Example 15 were used under the following conditions, and then the intensities of odors of the acid odors due to the armpit odor after the use were compared and evaluated according to evaluation criteria below by five expert panelists. Results are shown in Table 23 below. As obvious from Table 23, in the treatment by use of the aerosol powder according to Example 23, excellent acid odor inhibiting effect due to the masking action and the harmonizing action of the perfume composition for aerosol powder according to Example 21 was found in the gauze. On the contrary, in the treatment with the aerosol powder of Comparative Example 15, the acid odor was detected in the gauze even after the use.

### [Experimental conditions]

After taking a bath, an examinee having strong armpit odor was got to treat one armpit with the aerosol powder and to wear a shirt with previously washed gauzes attached on the places contacting with armpits directly. After 24 hours, the odors of the gauzes were evaluated by five expert panelists.

### [Evaluation criteria]

○: Better than non-applied.
△: Slightly better than non-applied. (Acid odor is slightly sensed.)
X: Equivalent to non-applied. (Acid odor is apparently sensed.)

### [Results of evaluation]

**Table 23**

| | Example 23 | Comparative Example 15 |
|---|---|---|
| Evaluation | ○ | △ |

## Claims

1. A deodorant perfume composition enabled to mask an acid odor, comprising one or more components selected from hydroxyalkynyl carboxylic acids having 5 to 8 carbon atoms and alkenyl carboxylic acids having 5 to 8 carbon atoms and one double bond comprising:
(I) at least one kind or more selected from compounds or essential oils of which aroma is excellent in both of the masking and harmonizing effects to the acid odor;
(II) at least one kind or more selected from compounds or essential oils of which aroma is excellent in the masking effect but neutral in the harmonizing effect to the acid odor.

2. The deodorant perfume composition according to claim 1, wherein the acid odor is 3-hydroxy-3- methylhexanoic acid and/or 3-methylhexenoic acid.

3. The deodorant perfume composition according to claim 1, wherein the component (I) is contained by 40 % by weight or more, components (I) and (II) are contained by 60 % by weight or more in sum total; further components are contained by less than 40 % by weight.

4. A deodorant composition, comprising the deodorant perfume composition according to any one of claims 1 to 3.

5. An external preparation composition, comprising the deodorant perfume composition according to any one of claims 1 to 3.

6. A method of inhibiting an acid body odor selected from hydroxyalkynyl carboxylic acids having 5 to 8 carbon atoms and alkenyl carboxylic acids having 5 to 8 carbon atoms and one double bond, by application of
(A) at least one kind or more selected from compounds or essential oils that are excellent in both of masking and harmonizing effects;
(B) at least one kind or more selected from compounds or essential oils that are excellent in the masking effect but neutral in the harmonizing effect.
(C) as needs arise, at least one kind or more selected from compounds or essential oils that exhibit neither the masking effect nor the harmonizing effect.

7. The method of inhibiting a body odor according to claim 6, wherein an acid odor is 3-hydroxy-3- methylhexanoic acid and/or 3-methylhexenoic acid.
